**Europäisches Patentamt**

**European Patent Office**

**Office européen des brevets**

(19)

(11) Veröffentlichungsnummer : **0 230 424**
**B1**

(12) # EUROPÄISCHE PATENTSCHRIFT

(45) Veröffentlichungstag der Patentschrift :
**09.11.88**

(21) Anmeldenummer : **86900105.7**

(22) Anmeldetag : **05.12.85**

(86) Internationale Anmeldenummer :
**PCT/EP 85/00671**

(87) Internationale Veröffentlichungsnummer :
**WO/8603390 (19.06.86 Gazette 86/13)**

(51) Int. Cl.⁴ : **A 61 B   5/04**, A 61 H   1/02

(54) **VERFAHREN ZUR STIMULATION DES MENSCHLICHEN VESTIBULARAPPARATES UND VORRICHTUNG HIERFÜR.**

(30) Priorität : **05.12.84 DE 3444777**

(43) Veröffentlichungstag der Anmeldung :
**05.08.87 Patentblatt 87/32**

(45) Bekanntmachung des Hinweises auf die Patenterteilung : **09.11.88 Patentblatt 88/45**

(84) Benannte Vertragsstaaten :
**AT BE CH DE FR GB IT LI LU NL SE**

(56) Entgegenhaltungen :
**FR-A- 1 076 232**
**US-A- 3 387 605**
**IEEE Transactions on Biomedical Engineering, vol. BME-28, no. 5, May 1981, New York (US), J.R. Tole et al.: "A microprocessor-controlled vestibular examination chair", pp. 390-395**

(73) Patentinhaber : **Westhofen, Martin, Dr.**
**Curschmannstrasse 12**
**D-2000 Hamburg 20 (DE)**

(72) Erfinder : **Westhofen, Martin, Dr.**
**Curschmannstrasse 12**
**D-2000 Hamburg 20 (DE)**

(74) Vertreter : **Richter, Werdermann & Gerbaulet**
**Neuer Wall 10**
**D-2000 Hamburg 36 (DE)**

## Beschreibung

Die Erfindung betrifft eine Vorrichtung zur Stimulation des menschlichen Vestibularapparates.

Bekannt ist die Prüfung der Funktion des menschlichen Vestibularapparates anhand des Spontan- und Blickrichtungs- und des Provokationsnystagmus sowie durch Drehprüfung. Auch ist bereits vorgeschlagen worden, zur Stimulation des menschlichen Vestibularapparates verschiedene Beschleunigungen untereinander zu kombinieren (IEEE Transactions von Biomedical Engineering, Band BME - 28, Nr. 5, Mai 1981).

Der Erfindung liegt daher die Aufgabe zugrunde, eine Vorrichtung zu schaffen, mit der es möglich ist, den menschlichen Vestibularapparat durch Beschleunigung des menschlichen Kopfes eines liegenden Patienten in mehreren Ebenen zu stimulieren.

Diese Aufgabe wird durch die im Patentanspruch gekennzeichneten Merkmale gelöst.

Die Reizung des Vestibularorgans hat grundsätzlich eine Erregung des Zentralnervensystems zur Folge. Reizabhängige Erregung des Zentralnervensystems läßt sich in Form sogenannter evozierter Potentiale registrieren. Hierzu werden vom Skalp des Patienten mit Hilfe von Elektroden elektrische Bio-Signale abgegriffen und aufgezeichnet. Zu diesem Zweck müssen zahlreiche identische Einzelstimuli in kurzer zeitlicher Abfolge apliziert werden (Averaging-Technik).

Mit der erfindungsgemäßen Vorrichtung ist eine magnetgesteuerte Linear- und Drehpendeleinheit zur Stimulation des menschlichen Vestibularapparates geschaffen. Die Stimulationsvorrichtung bewegt den menschlichen Kopf in liegender Position des Patienten in Form einer Drehpendelbeschleunigung um eine horizontale Achse und einer Linearbeschleunigung in senkrechter Richtung. Drehpendel- und Linearpendelbewegungen sind unabhängig voneinander und simultan durchführbar.

Die Größe der Beschleunigung und Amplitude, der zeitliche Ablauf sowie der Drehpunkt der Bewegung bzw. Bewegungen sind veränderbar.

Mit der Stimulationsvorrichtung werden folgende Vorteile erreicht:

- Beliebige Kombination einer Linear- und einer Drehbeschleunigung mit unabhängiger Steuerung beider Einzelbewegungen.
- Freie Wählbarkeit der Form und des zeitlichen Ablaufs der Drehpendel- und der Linearpendelbewegung.
- Liegende Position des Patienten.
- Beliebige Wiederholbarkeit der einzelnen Stimulusmanöver.
- Die Vorrichtung arbeitet geräuscharm; alle mit der Vorrichtung durchgeführten Funktionen sind geräuscharm.

Vorteilhafte Ausgestaltungen und Weiterbildungen der Erfindung sind in den Unteransprüchen gekennzeichnet.

Ein Ausführungsbeispiel der Erfindung wird nachstehend anhand der Zeichnung näher erläutert. Es zeigen

Fig. 1 die Vorrichtung mit den Antrieben zur Erzeugung von Drehpendel- und Linearpendelbewegungen in einer Seitenansicht,

Fig. 2 den Antrieb für die Drehpendelbewegung der Antriebsachse in einer Ansicht und

Fig. 3 eine Draufsicht auf den die Kopfmulde tragenden Rahmen der Vorrichtung von der Seite der Antriebsachse her.

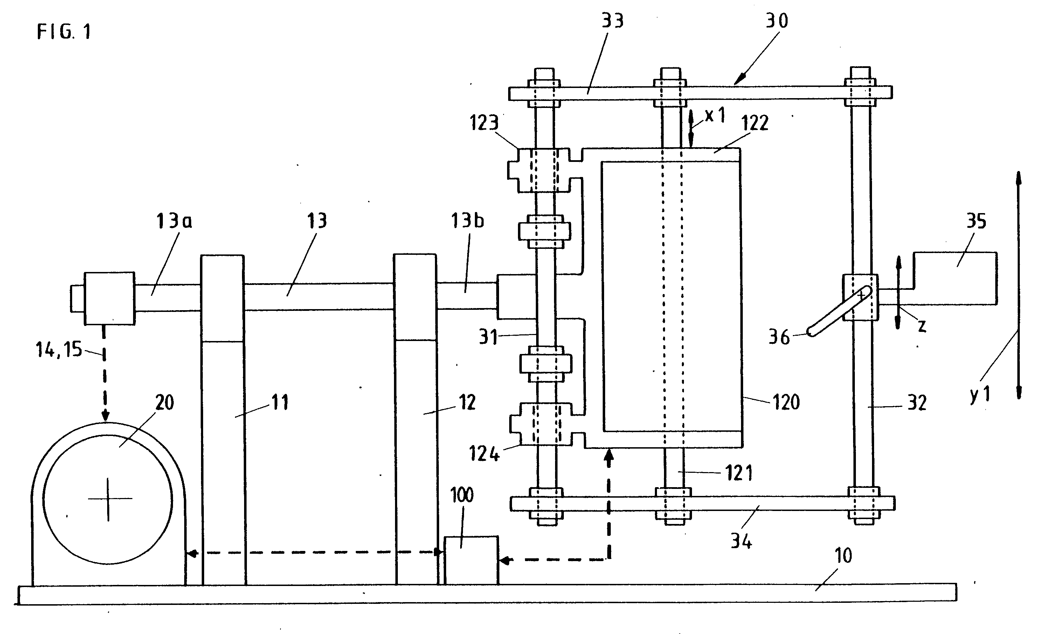

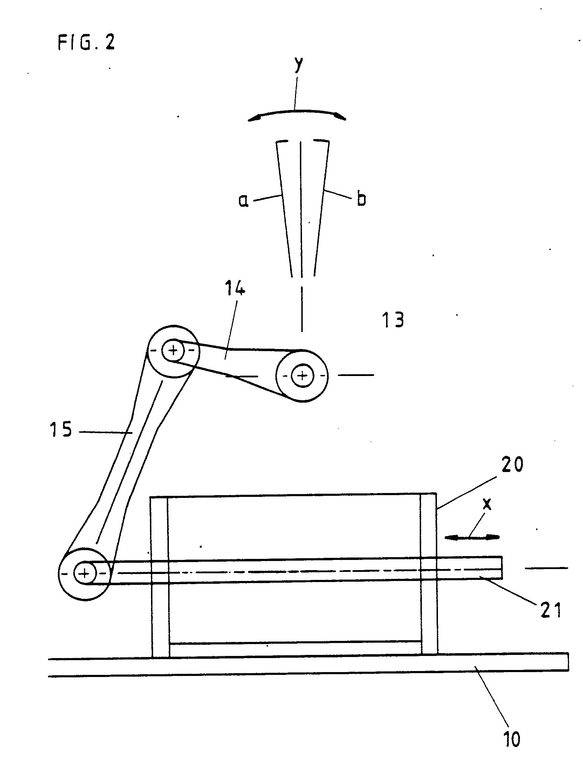

Die Vorrichtung zur Stimulation des menschlichen Vestibularapparates besteht nach Fig. 1 aus einer Grundplatte 10, die Lager 11, 12 für eine waagerechte Antriebsachse 13 trägt. Diese Antriebsachse 13 steht zur Erzeugung einer Drehpendelbewegung mit einem Antrieb in Verbindung, der aus einer Kurbel 14 mit einer Pleuelstange 15 und einem Elektro-Umkehrhubmagneten 20 besteht, der ebenfalls auf der Grundplatte 10 angeordnet ist. An das freie Ende des stabförmigen Ankers 21 des Umkehrhubmagneten 20 ist die Pleuelstange 15 mit ihrem freien Ende angelenkt, so daß bei einer Bewegung des Ankers 21 in Pfeilrichtung X die Antriebsachse 13 um ihre Längsachse wechselseitig gedreht wird, so daß eine Drehpendelbewegung erzielt wird (Fig. 2). Diese Drehpendelbewegung, die in Pfeilrichtung Y erfolgt, ist begrenzt durch die in Fig. 2 angedeuteten Umkehrpunkte a und b, d. h., daß in diesen Endbereichen a und b die jeweilige Umkehrung der Bewegung erfolgt.

Der Umkehrhubmagnet 20 besteht aus zwei in der Zeichnung nicht dargestellten einzelnen Spulen, die den Anker 21 des Umkehrhubmagneten 20 in entgegensetzte Richtungen ziehen. Durch wechselweise Anregung der Spulen wird dann die für die Erzeugung der Drehpendelbewegung der Antriebsachse 13 erforderliche Bewegung des Ankers 21 des Umkehrhubmagneten 20 erreicht.

Während an dem freien Ende 13a der Antriebsachse 13 die Pleuelstange 15 angelenkt ist, trägt die Antriebsachse 13 an ihrem anderen Ende 13b einen weiteren Elektro-Umkehrhubmagneten 120, der entsprechend dem Umkehrhubmagnet 20 ausgebildet und ebenfalls mit zwei in der Zeichnung nicht dargestellten Spulen versehen ist, mit denen der stabförmige Anker 121 des Umkehrhubmagneten 120 wechselweise in Pfeilrichtung X1 bewegbar ist.

Der Anker 121 des Umkehrhubmagneten 120 ist mit einem senkrechtstehenden Rahmen 30 fest verbunden, der eine quadratische oder rechteckförmige Form aufweist und aus zwei zu beiden Seiten des Ankers 121 und parallel zu diesem angeordneten Führungsstangen 31, 32 besteht, die über waagerechte Stäbe 33, 34 fest miteinander verbunden sind (Fig. 1). An diesen waagerechten Stäbe 33, 34 ist der Anker 121 des Umkehrhubmagneten 120 mit seinen beiden Enden befestigt.

Die Führungsstange 32 des Rahmens 30 trägt eine muldenförmige Kopfauflage 35, die nachstehend mit Kopfmulde bezeichnet ist. Diese Kopfmulde 35 ist an der Führungsstange 32 längsverschieblich gehalten und mittels einer entsprechend ausgebildeten Feststelleinrichtung 36, die als Feststellschraube od. dgl. ausgebildet sein kann, in jeder Stellung an der Führungsstange 32 feststellbar. Die Verschiebbarkeit der Kopfmulde 35 erfolgt in Pfeilrichtung Z (Fig.1).

Die der Antriebsachse 13 zugekehrte Führungsstange 31 des Rahmens 30 dient gleichzeitig zur Aufnahme und Führung von Gleithülsen 123, 124, die an dem Tragrahmen 122 des Umkehrhubmagneten 120 angeformt sind. Diese Gleithülsen 123, 124 bestehen aus Kunststoffen, insbesondere aus Formstücken aus Polytetrafluoräthylen. Werden die Spulen des Umkehrhubmagneten 120 erregt, so wird der Anker 121 des Umkehrhubmagneten 120 zusammen mit dem Rahmen 33 und der Kopfmulde 35 in Pfeilrichtung Y1 in der Senkrechten bewegt, d. h. es wird eine senkrechte Linearbewegung des Rahmens 30 mit der Kopfmulde 35 erreicht. Die Führung des Rahmens 30 erfolgt mittels der Gleithülsen 123, 124 an dem Tragrahmen 122 des Umkehrhubmagneten 120, da die Führungsstange 31 des Rahmens 30 in diesen Gleithülsen 123, 124 geführt ist. Dadurch, daß der Umkehrhubmagnet 120 fest mit der Antriebsachse 13 verbunden ist und diese Antriebsachse einer Drehpendelbewegung um ihre Längsachse unterliegt, wird der in der Kopfmulde 35 liegende Kopf eines eine liegende Position einnehmenden Patientens einer Drehpendelbeschleunigung um eine horizontale Achse und einer Linearpendelbeschleunigung in senkrechter Richtung unterworfen.

Die beiden Umkehrhubmagneten 20, 120 werden über an sich bekannte programmierbare Leistungsnetzteile gesteuert, die z. B. über einen Mikroprozessor ansteuerbar sind. Die Steuereinrichtung für die beiden Umkehrhubmagneten 20, 120 ist in Fig. 1 bei 100 angedeutet. Die beiden Umkehrhubmagneten 20, 120 können somit durch gesteuerte elektrische Ansteuerung beliebige Pendelbewegungsabläufe der Kopfmulde 35 bewirken. Für die Linearbewegung ist vorgesehen ein Verschieben des Rahmens 30 mit der Kopfmulde 35 in der Senkrechten, um eine Distanz von etwa 5 cm zwischen den jeweiligen Umkehrpunkten.

Die beiden parallel zum Anker 121 des Umkehrhubmagneten 120 verlaufenden Führungsstangen 31, 32 des Rahmens 30 sollen ein Verkanten des Ankers 121 des Umkehrhubmagneten 120 bei einer Belastung der Kopfmulde 35 verhindern. Eine der beiden Führungsstangen 31, 32 läßt sich in ihrer Länge verändern, so daß bei einer Belastung der Kopfmulde 35 ein einwandfreies Gleiten der Führungsstange 31 des Rahmens 30 in den Gleithülsen 123, 124 des Tragrahmens 122 des Umkehrhubmagneten 120 gewährleistet ist. Wie Fig. 1 zeigt, ist der komplette, der Linearbewegung dienende Geräteteil, bestehend aus dem Umkehrhubmagneten 120, dem Rahmen 30 mit den beiden Führungsstangen 31, 32, den Gleithülsen 123, 124 an dem Tragrahmen 122 des Umkehrhubmagneten 120 sowie der Kopfmulde 35 auf der horizontal liegenden Antriebsachse 13 aufgeflanscht. Anstelle des voranstehend beschriebenen und in Fig. 2 dargestellten, aus der Kurbel 14 und der Pleuelstange 15 bestehenden Antriebes können auch andere an sich bekannte Antriebe Verwendung finden. Wesentlich ist, daß die Bewegung des Ankers 21 des Umkehrhubmagneten 20 in eine Drehpendelbewegung der Antriebsachse 13 umgesetzt wird. Die Dimensionierung des Umkehrhubmagneten 20 und der Kurbel 14 mit der Pleuelstange 15 ist so ausgelegt, daß ein Pendelwinkel von 12° zwischen den Umkehrpunkten a und b erreicht wird (Fig. 2). Um die Antriebsachse 13 mit ihrem den Umkehrhubmagneten 120 tragenden Ende 13b im Bereich der Führungsstange 31 des Rahmens 30 in den Rahmen einführen zu können, kann die Führungsstange 31 mittig mit einer Durchbohrung versehen sein, durch die das freie Ende 13b der Antriebsachse 13 hindurchgeführt ist. Der Rahmen 30 ist mittig zur Antriebsachse 13 an dieser angeordnet.

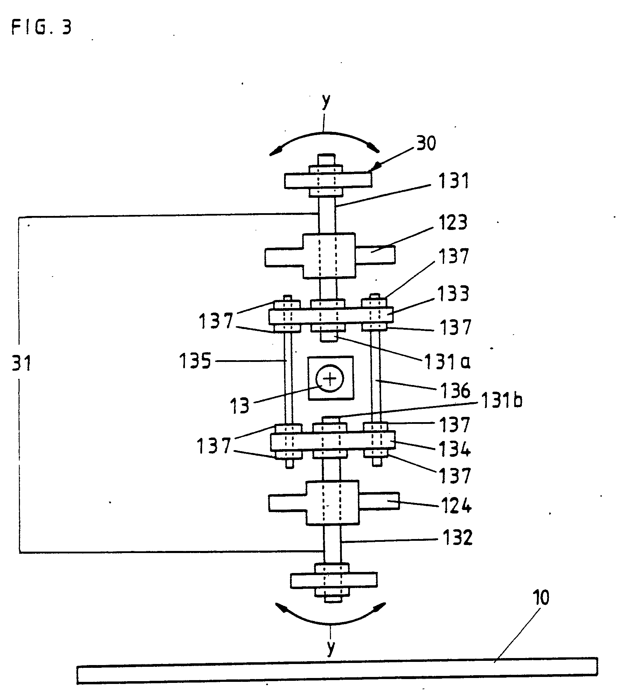

Wie Fig. 3 zeigt, kann die Führungsstange 31 zum Hindurchführen des Endes 13b der Antriebsachse 13 auch eine anderweitige Ausgestaltung aufweisen. Nach Fig. 3 besteht die Führungsstange 31 des Rahmens 30 aus zwei miteinander fluchtenden, senkrechten Führungsstangenabschnitten 131, 132, deren einander zugekehrte freie Enden 131a, 132a oberhalb und unterhalb der Antriebsachse 13 enden. An ihren freien Enden 131a, 132a tragen die Führungsstangenabschnitte waagerechte Tragstäbe 133, 134, die über senkrechte Verbindungsstäbe 135, 136 miteinander verbunden sind. Auf den Führungsstangenabschnitten 131, 132, und zwar oberhalb der Tragstäbe 133, 134, sind die Gleithülsen 123, 124 des Tragrahmens 122 des Umkehrhubmagneten 120 angeordnet.

Die Verbindungsstäbe 135, 136 sind mit Außengewinden versehen und mittels bei 137 in Fig. 3 angedeuteten Muttern an den Tragstäben 133, 134 gehalten. Aufgrund dieser Ausgestaltung besteht die Möglichkeit, durch entsprechendes Verstellen der Muttern den Abstand der beiden Führungsstangenabschnitte 131, 132 voneinander zu verändern, d. h. die beiden Verbindungsstäbe 135, 136 sind spindelartig ausgebildet. Aufgrund dieser Verstellmöglichkeit läßt sich letztlich die Gesamtlänge der Führungsstange 31 verändern, so daß bei einer Belastung der Kopfmulde 35 ein einwandfreies Gleiten der Führungsstange 31 in den beiden Gleithülsen 123, 124 des Tragrahmens 122 des Umkehrhubmagneten 120 gewährleistet ist.

Die Gesamtvorrichtung, d. h. die Antriebsachse 13 mit ihren beiden Lagern 11, 12 ist auf der Grundplatte 10 angeordnet, die als Siebdruckplatte ausgebildet sein kann. Alle Teile der Vorrichtung können mittels eines Gehäuses, welches auf der Grundplatte 10 lösbar befestigt ist, abgedeckt sein. Das Gehäuse besteht aus Aluminium, Kunststoffen oder anderen geeigneten Werkstoffen und ist mit Durchbrechungen für das Hindurchführen der bewegten Teile versehen.

Zusätzliche Blenden verschließen Öffnungen in dem Gehäuse, um ein Verletzen von Personen bei Betrieb der Vorrichtung auszuschließen.

Die Steuerung der Umkehrhubmagnete 20, 120 erfolgt über die Steuereinrichtung 100. Die Ableitung und Registrierung der evozierten Potentiale des Patienten entspricht der bekannten Technik zur Gewinnung akustisch und optisch evozierter Potentiale.

**Patentansprüche**

1. Vorrichtung zur Stimulation des menschlichen Vestibularapparates, die aus einer auf einer Grundplatte (10) in Lagern (11, 12) gehaltenen waagerechten Antriebsachse (13), deren eines Ende (13a) über einen Antrieb, wie eine Kurbel (14) mit einer Pleuelstange (15) od. dgl. verbunden ist, die zur Erzielung einer Drehpendelbewegung der Antriebsachse (13) an dem freien Ende des stabförmigen Ankers (21) eines ersten auf der Grundplatte (10) angeordneten Elektro-Umkehrhubmagneten (20) angelenkt ist, und zur Erzielung einer senkrechten Linearbewegung aus einem an dem anderen freien Ende (13b) der Antriebsachse (13) befestigten zweiten Elektro-Umkehrhubmagneten (120), dessen stabförmiger Anker (121) mit einem senkrechtstehenden und etwa quadratischen oder rechteckförmigen Rahmen (30) aus zwei senkrechten, über waagerechte Stäbe (33, 34) miteinander verbundenen Führungsstangen (31, 32) verbunden ist, und aus der an der der Antriebsachse (13) abgekehrten Seite des Rahmens (30) liegenden Führungsstange (32) verschieblich gehalten und in jeder Stellung an der Führungsstange (32) feststellbaren (36) muldenförmigen Kopfauflage (35) besteht, wobei der mit der Antriebsachse (13) verbundene Tragrahmen (123) des zweiten Elektro-Umkehrhubmagneten (120) zwei im Abstand voneinander angeordnete Gleithülsen (123, 124) aufweist, in denen die auf der der Antriebsachse (13) zugekehrten Seite des Rahmens (30) liegende Führungsstange (31) geführt ist.

2. Vorrichtung nach Anspruch 1, dadurch gekennzeichnet, daß die der Antriebsachse (13) zugekehrte Führungsstange (31) des Rahmens (30) mit einer Durchbohrung zum Hindurchführen des den zweiten Elektro-Umkehrhubmagneten (120) tragenden Endes (13b) der Antriebsachse (13) versehen ist.

3. Vorrichtung nach Anspruch 1, dadurch gekennzeichnet, daß die der Antriebsachse (13) zugekehrte Führungsstange (31) des Rahmens (30) aus zwei miteinander fluchtenden Führungsstangenabschnitten (131, 132) besteht, deren einander zugekehrten freien Enden (131a, 132a) oberhalb und unterhalb der Antriebsachse (13) enden und die an ihren einander zugekehrten freien Enden (131a, 132a) waagerechte Tragstäbe (133, 134) aufweisen, die zur Veränderung des Abstandes der beiden Führungsstangenabschnitte (131, 132) voneinander über senkrechte Verbindungsstäbe (135, 136) miteinander verbunden sind, die Gewinde tragen und mittels Muttern (137) an den Tragstäben (133, 134) gehalten sind.

4. Vorrichtung nach Anspruch 1 bis 3, dadurch gekennzeichnet, daß zur Steuerung der Elektro-Umkehrhubmagnete (20, 120) programmierbare, über Mikroprozessoren ansteuerbare Leistungsnetzteile (100) vorgesehen sind.

5. Vorrichtung nach Anspruch 1 bis 4, dadurch gekennzeichnet, daß die Antriebsachse (13) mit ihrem Elektro-Umkehrhubmagneten (20) und mit dem zweiten Elektro-Umkehrhubmagneten (120) mit dem Rahmen (30) in einem Gehäuse aus Aluminium, Kunststoff oder anderen geeigneten Werkstoffen unter Freilassung der Kopfmulde (35) angeordnet ist.

6. Vorrichtung nach Anspruch 4, dadurch gekennzeichnet, daß die Elektro-Umkehrhubmagnete (20, 120) unabhängig voneinander sowie simultan ansteuerbar sind, wobei über den Mikroprozessor die Größe der Beschleunigung und der Amplitude und der zeitliche Ablauf veränderbar sind und der Drehpunkt der Bewegungen veränderbar ist.

**Claims**

1. Device for stimulating the human vestibular apparatus which consists of a horizontal driving axle (13) fixed on a base plate (10) and running on bearings (11, 12), the one end (13a) of which being connected with a connecting rod (15) via a drive, such as a crank (14) or the like, which, for the purpose of achieving a rotary pendulum movement of the driving axle (13), is articulated to the free end of the rod-shaped anchor (21) of a first electric reversing hoisting magnet (20) which is arranged on the base plate (10), and, for the purpose of achieving a vertical linear movement, consists of a second electric reversing hoisting magnet (120) mounted to the other free end (13b) of the driving axle (13), the rod-shaped anchor (121) of which is connected with a vertically disposed and in general square or rectangular frame (30) of two vertical guide rods (31, 32) joined via horizontal rods (33, 34), and of the trough-shaped head support (35) displacably attached on the side of the frame (30) opposite the driving axle (13) and which can be locked (36) in each position to the guide rod (32), the supporting frame (123) of the second electric reversing hoisting magnet (120) joined with the driving axle (35) being provided with two spaced slide sleeves (123, 124) in which the guide rod (31) is guided, which is arranged on the side of the frame (30) directed to the driving axle (13).

2. Device according to claim 1, characterized in that the guide rod (31) of the frame (30) directed towards the driving axle (13) is provided with a perforation through which the end (13b) of the driving axle (13) bearing the second electric reversing hoisting magnet (120) can be passed.

3. Device according to claim 1, characterized in that the guide rod (31) of the frame (30) directed towards the driving axle (13) consists of two aligned guide rod portions (131, 132), the two free ends (131a, 132a) of which facing towards each other end up above and under the driving axle (13) and which have horizontal supporting rods (133, 134) at their free ends facing towards each other, which, for the purpose of changing the space between the two guide rod portions (131, 132), are connected with each other via vertical connecting rods (135, 136) threaded and held to the supporting rods (133, 134) by means of nuts (137).

4. Device according to claims 1 to 3, characterized in that, for the purpose of control of the electric reversing hoisting magnets (20, 120), programmable microprocessor controlled power supply units (100) are provided.

5. Device according to claims 1 to 4, characterized in that the driving axle (13) with its electric reversing hoisting magnet (20) and with the second electric reversing hoisting magnet (120) is arranged with the frame (30) in a casing of aluminium, plastic or other appropriate materials leaving the trough-shaped head support (35) clear.

6. Device according to claim 4, characterized in that the electric reversing hoisting magnets (20, 120) can be controlled separately or simultaneously, whereby the parameters of acceleration and amplitude and timing and the pivot of motion can be modified by means of the microprocessor.

**Revendications**

1. Dispositif pour stimuler l'appareil vestibulaire de l'homme qui se compose d'un arbre de commande (13) maintenu horizontalement dans des paliers (11, 12) sur une plaque d'assise (10), arbre dont l'une des extrémités (13a) est reliée par une commande, telle qu'une manivelle (14), à une bielle (15) ou équivalent qui, pour obtenir un mouvement oscillant rotatif de l'arbre de commande (13), est articulée à l'extrémité libre de l'armature en forme de barre (21) d'un premier électro-aimant de levage réversible (20) placé sur la plaque d'assise (10) et, pour obtenir un mouvement linéaire vertical, d'un second électro-aimant de levage réversible (120) fixé à l'autre extrémité libre (13b) de l'arbre de commande (13), dont l'armature en forme de barre (121) est reliée à un cadre (30) perpendiculaire, de forme à peu près carrée ou rectangulaire se composant de deux tiges conductrices verticales (31, 32) reliées l'une à l'autre par des barres horizontales (33, 34), et du support pour la tête (35) en forme d'auge maintenu de manière coulissante sur la tige conductrice (32) située sur le côté du cadre (30) qui n'est pas tourné vers l'arbre de commande (13) et pouvant être bloqué (36) sur la tige conductrice (32) à n'importe quelle position, le cadre porteur (123) du deuxième électro-aimant de levage réversible (120), qui est relié à l'arbre moteur (13), présentant deux douilles coulissantes (123, 124) placées à une certaine distance l'une de l'autre dans lesquelles la tige conductrice (31) située sur le côté du cadre (30) tourné vers l'arbre de commande (13) est guidée.

2. Dispositif selon la revendication 1, caractérisé en ce que la tige conductrice (31) du cadre (30) qui est tournée vers l'arbre de commande (13) est pourvue d'une forure pour faire traverser l'extrémité (13b) de l'arbre de commande (13) qui porte le deuxième électro-aimant de levage réversible (120).

3. Dispositif selon la revendication 1, caractérisé en ce que la tige conductrice (31) du cadre (30) qui est tournée vers l'arbre de commande (13) se compose de deux tronçons de tige conductrice (131, 132) alignés l'un sur l'autre dont les extrémités libres (131a, 132a) tournées l'une vers l'autre se terminent au-dessus et au-dessous de l'arbre de commande (13) et qui présentent, à leurs extrémités libres (131a, 132a) tournées l'une vers l'autre des barres porteuses horizontales (133, 134) qui sont reliées l'une à l'autre par des barres de jonction verticales (135, 136) pour faire varier l'écart entre les deux tronçons de tige conductrice (131, 132), barres de jonction qui sont filetées et qui sont maintenues sur les barres porteuses (133, 134) au moyen d'écrous (137).

4. Dispositif selon les revendications 1 à 3, caractérisé en ce que des blocs d'alimentation de puissance (100), pouvant être excités par des microprocesseurs, sont prévus pour la commande des électro-aimants de levage réversibles (20, 120).

5. Dispositif selon les revendications 1 à 4, caractérisé en ce que l'arbre moteur (13) avec son électro-aimant de levage réversible (20) et avec le deuxième électro-aimant de levage réversible (120) avec le cadre (30) est placé dans un boîtier en aluminium, plastique ou en une autre matière appropriée en laissant libre le support pour la tête en forme d'auge (35).

6. Dispositif selon la revendication 4, caractérisé en ce que les électro-aimants de levage réversibles (20, 120) peuvent être excités indépendamment l'un de l'autre ainsi que simultanément, l'ampleur de l'accélération et de l'amplitude et le déroulement dans le temps pouvant être variés par le microprocesseur et le point de rotation des déplacements étant variable.

FIG. 1

0 230 424

FIG. 2

FIG. 3